# EUROPEAN PATENT APPLICATION

(11) **EP 4 098 185 A1**
(43) Date of publication of application: **07.12.2022**
(21) Application number: 21711968.4
(22) Date of filing: 28.01.2021
(51) Int. Cl.: A61B 5/08, A61B 5/296, A61B 5/28, A61B 5/01, A61B 5/00

(54) **SYSTEM, METHOD, PORTABLE DEVICE, COMPUTER APPARATUS AND COMPUTER PROGRAM FOR MONITORING, CHARACTERISATION AND ASSESSMENT OF A USER'S COUGH**

(30) Priority: 29.01.2020 PT 2020116079
(71) Applicant: Cough Monitoring Medical Solutions, Lda., 2825-182 - Caparica (PT)
(72) Inventor: JACINTO TECELÃO, Diogo Bernardo, 2970-577 Sesimbra (PT); NETO MAGALHÃES FERREIRA ANDRADE, Miguel, 2120-050 Salvaterra de Magos (PT); DIAS LOPES, Alexandra Sofia, 2620-047 Olival Basto (PT); CAETANO VALADAS, Filipe André, 2795-042 Linda-a-Velha (PT); ALMEIDA E SOUSA DE ALMADA LOBO, Sara Beatriz, 2800-043 Almada (PT)
(74) Representative: Monteiro Alves, Inês
(86) International application number: PCT/PT2021/050002
(87) International publication number: WO 2021/154105

(57) **Abstract**

The invention concerns a system for monitoring, characterising and evaluating a user's cough comprising a sensory instrumentation unit (2), including at least one abdominal contraction sensor (3) and at least one acoustic sensor (4), wherein the acoustic sensor (4) is triggered only when abdominal contractions are detected; a microcontroller (1); at least one data flow unit; and a data processing and analysis unit for cough assessment (5).

The invention solves the problem of under-assessment of cough in medical practice, allowing to objective analyse the cough, contributing to the medical diagnosis by establishing parameterized cough patterns, involving the frequency, type of cough, the user's body position and other data regarding events which potentially cause cough. The invention also protects the user's privacy, as acoustic sensory data are only recorded when there are abdominal contraction events.

## Description

### Technical Field

The present invention concerns systems to support medical diagnosis, specifically the monitoring, characterisation and evaluation of a user's cough.

The present invention also refers to a method, a portable device, a computer apparatus, a computer program and a means of reading by a computer apparatus for monitoring, characterising and evaluating a user's cough using such a system.

### Background Art

Cough is a symptom which has already been experienced by all human beings and it is an essential mechanism for the protection of the body. It is the symptom most frequently mentioned by patients when seeking medical advice and the most common reason for visits to primary health care. Furthermore, persistent cough, i.e. cough lasting more than three weeks, is the most common reason for visits to respiratory specialists. Finally, the prevalence of chronic cough, i.e. a cough lasting more than three months, totals 9.6% worldwide and, more specifically, 12.5% in Europe and 11.0% in the United States. It is important to note that the increase of cough's frequency and intensity can reduce the quality of life by interfering with breathing, eating and sleeping, impairing the health and quality of life of the patient.

There are several possible causes for acute, persistent and chronic cough, namely upper respiratory tract infections, asthma, chronic obstructive pulmonary disease, gastroesophageal reflux, pneumonia, heart failure, lung cancer, post-nasal drip, among others. Cough can be the first obvious sign of airway or lung disease, when it represents more than a defence mechanism. Due to its persistence and distinctive characteristics, cough becomes a useful indicator of a disease which is potentially developing or progressing. In this way, cough is an extremely important symptom in its underlying diseases.

Thus, a complete and comprehensive assessment of the patient's cough is fundamental to identify and treat its underlying cause, since each individual may have different factors contributing to their status. This assessment should include the frequency of the cough; the type of the cough, i.e., whether the cough is productive and/or dry and if it presents other characteristics, such as whether it is accompanied by wheezing; and possible patterns associated with the frequency and type of the cough, for example, whether the cough has a pattern of nocturnal predominance, and patterns associated with possible events which may cause the cough, for example, whether the decubitus position triggers cough episodes.

Cough's characteristics and patterns vary according to the disease. In the case of asthma exacerbation, cough episodes occur mainly during the night and early morning. Another example refers to the case of exercise-induced asthma, where one of the most significant symptoms is coughing during or after periods of physical exertion. In the case of gastroesophageal reflux, cough episodes may precipitate gastroesophageal reflux, due to contractions of the abdominal muscles and the resulting increase in intra-abdominal pressure, which will contribute to the rise of the gastric juice. On the other way, reflux episodes may trigger cough through a reflex mechanism resulting from the rise of the gastric juice through the esophagus and consequent inflammation and irritation of the upper airway (and not due to an existing respiratory disease). In the case of cough triggered by reflux, the occurrence of cough episodes may be heightened when the patient is lying down due to the easier rise of the gastric juice.

However, despite the importance of cough, it is considered a highly subjective symptom for patients. For example, some patients may consider their cough to be highly frequent, while other patients would not judge the same cough in the same way. In the case of a day-time cough, the description of its predominance is extremely reliant on the patient's memory and subjectivity. In the case of a night-time cough, cough episodes are rarely recalled accurately. Furthermore, patients often misclassify their type of cough and a forced cough in consultation does not always reflect the spontaneous coughs of the patient and does not reflect its progression over time. All of this contributes to the high subjectivity and difficulty of cough evaluation in clinical practice, which often causes the health professional's analysis to be very difficult.

There is a known long-standing unmet medical need of doctors and healthcare providers for a reliable and portable device, capable of being used without changing the ordinary rhythm of daily life, to provide a comprehensive quantitative and qualitative assessment of the patient's cough, in order to allow early detection of the several conditions associated to and underlying cough, to consequently improve diagnosis and treatment plans.

Currently there are no cough monitoring tools available for the clinical practice, which puts doctors in a situation with incomplete and often subjective and inaccurate information to diagnose their patients.

The state of the art reveals some devices which aim to complement medical diagnosis based on cough events.

The international patent application WO2013142908A1 ((Univ. Queensland) 3 March 2013, pages 6 to 8 of the description and abstract) reveals a device which detects and characterises cough types based on audio records. Based on the classification of cough events, cough can be characterised, for example, as dry cough or productive cough, and eventually be associated with a disease. The device may contain other sensors to monitor other user data, such as motion sensors and EEG electroencephalography sensors. However, this device presents limitations regarding the methods based on cough classification linked solely to audio signals, as the results of cough classification may be insufficient for a more complete characterisation and assessment of its user. Although the device may include several additional sensors, these are not used to detect cough patterns. This device records audio continuously, recording the user's private conversations on a recurring basis, without preserving the user's right to privacy.

In patent application CN108294756A ((First Affiliated Hospital of Guangzhou Medical Univ. et al.) 20 July 2018, abstract), it is revealed a device which has several sensors, for example a microphone, an electromyography sensor with a three-electrode configuration and an accelerometer, to detect cough events and quantify their intensity. The device revealed in this reference uses the electromyography sensors and the accelerometer as auxiliary to the microphone in detecting cough events of the user. However, this device does not characterise the type of cough, for example, as dry or productive, nor does it establish coughing patterns or provide information which may be useful to help diagnose its user's clinical status. This device records audio continuously, which may record its user's conversations, and a computer program can later analyse the record and delete the parts which contain voice.

Additionally, and in contrast to the present invention, both the device revealed in WO2013142908A1 and the device revealed in CN108294756A have the problem of continuously recording and analysing the user's audio signals, resulting in an issue of its user's loss of privacy.

Therefore, there is a need to develop devices which perform a more objective and comprehensive cough assessment, namely, which establish cough patterns and are able to extract information with relevant content to help a physician to establish clinical diagnoses more efficiently and accurately. Additionally, there is a need to develop devices which preserve the users' privacy and their interpersonal interactions, both in private and public spaces, when collecting sound signals to perform cough characterisation.

The present application has as main objectives: (1) to empower doctors and other healthcare professionals with a portable, easy-to-use tool which allows the monitoring and objectively analysis of a user's cough; and (2) to provide users to which the device can be applied through an easy-to-use system which does not hinder their daily routines or privacy, combined with a cough monitoring method that will allow to provide healthcare professionals with more effective tools for early and efficient diagnosis.

The present invention was developed to perform a full characterisation and assessment of the cough, through the monitoring and automatic detection of several parameters necessary to assist in reaching an accurate diagnosis of the user, for example:
- the frequency of the cough, specifically the number of times the user coughs and the distribution of cough events over time;
- the type of cough, specifically whether the cough is dry, productive and/or if it presents
- other characteristics, such as whether it is accompanied by wheezing; and
- of cough patterns, involving the frequency and type of cough, the position and body movements of the user, and through other supplementary informative data of events which may cause cough.

Is is not known from the state of the art a portable device or system performing a full cough assessment, with the possibility of providing reports comprising an analysis of several parameters related to the cough's type and frequency and linking it to patterns which are important for diagnosis, namely by including additional sensors and supplementary devices which provide additional information on events that may trigger cough.

Therefore, the system for monitoring, characterisation and evaluation of a user's cough described and claimed in the present invention in appreciation, performs a complete evaluation of the user's cough, and will facilitate and accelerate the diagnosis of their underlying diseases, and can be used as a complementary diagnostic tool by the healthcare professional.

In addition to its usefulness as a tool for clinical practice, the capabilities of the present invention in appreciation also allow it to be used in the field of medical research, in particular to study the effect of certain drugs or pathologies on cough.

### Brief Description of Drawings

In order to promote an understanding of the principles according to the embodiments of the present invention, reference will be made to the embodiments illustrated in the figures and the language used to describe them. In any case, it should be understood that there is no intention to limit the scope of the present invention to the content of the figures. Any subsequent changes or modifications of the inventive features illustrated herein and any additional applications of the principles and embodiments of the invention illustrated, which would normally occur for a person skilled in the art having possession of this description, are considered within the scope of the claimed invention.
**Fig.1**

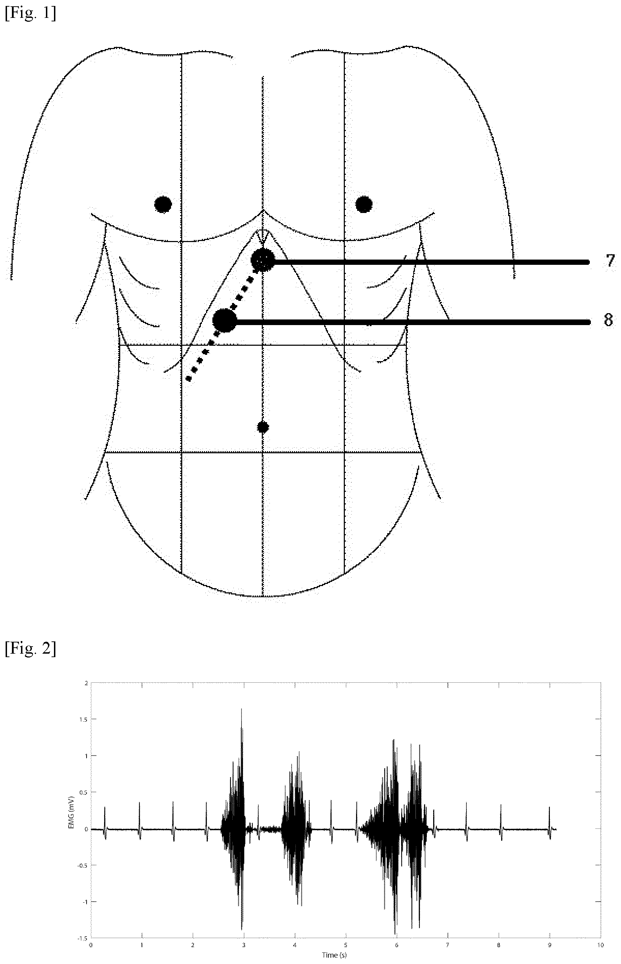

   [fig.1] illustrates the contact of the electromyography surface sensors on a user's body in an embodiment of the system of the present invention;
**Fig.2**
   [fig.2] illustrates the sensorial signals collected by a surface electromyography sensor in an embodiment of the system of the present invention;
**Fig.3**

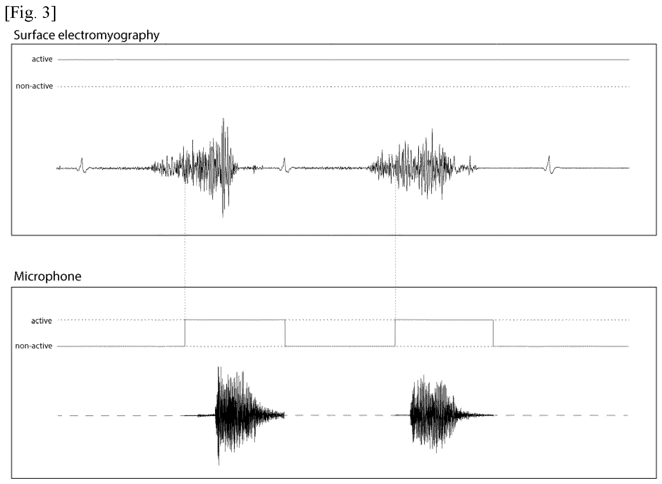

   [fig.3] illustrates the sensorial signals collected by the acoustic sensor, when it is activated by the microphone activation unit based on the surface electromyography sensor, in an embodiment of the system of the present invention;
**Fig.4**

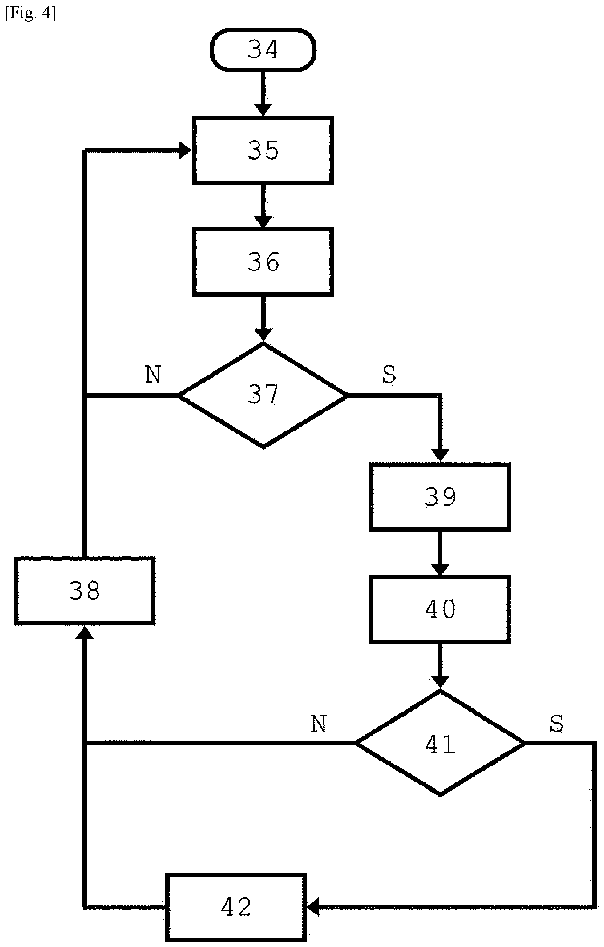

   [fig.4] illustrates the acoustic sensor activation algorithm through the detection of abdominal contraction events by the abdominal contraction sensor;
**Fig.5**

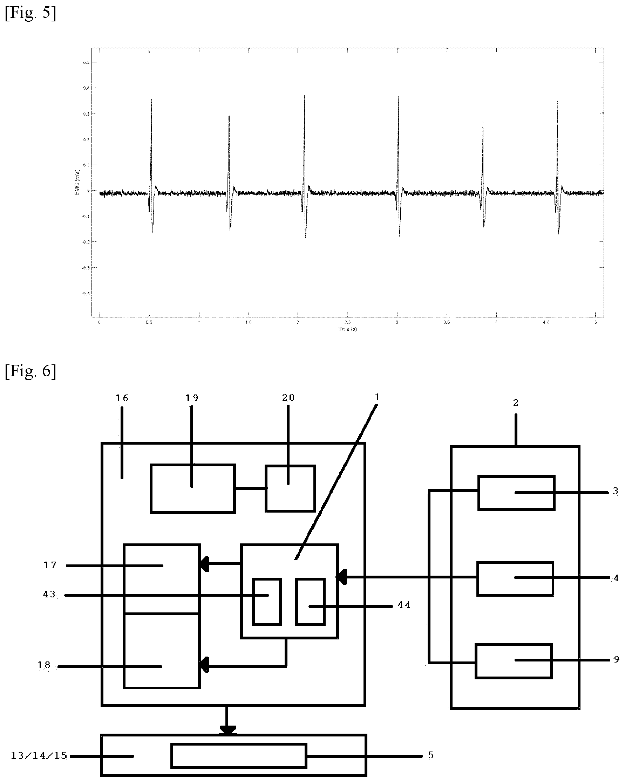

   [fig.5] illustrates the heartbeat signals collected by the surface electromyography sensor, in an embodiment of the system of the present invention;
**Fig.6**
   [fig.6] illustrates a first embodiment of the system according to the present invention;
**Fig.7**

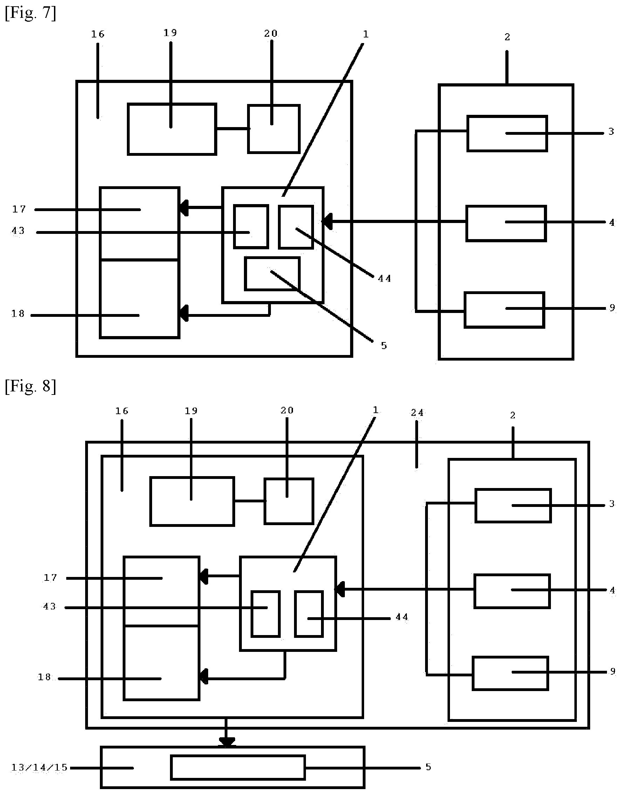

   [fig.7] illustrates a second embodiment of the system according to the present invention;
**Fig.8**
   [fig.8] illustrates a first embodiment of the system according to the present invention incorporated in a patch;
**Fig.9**

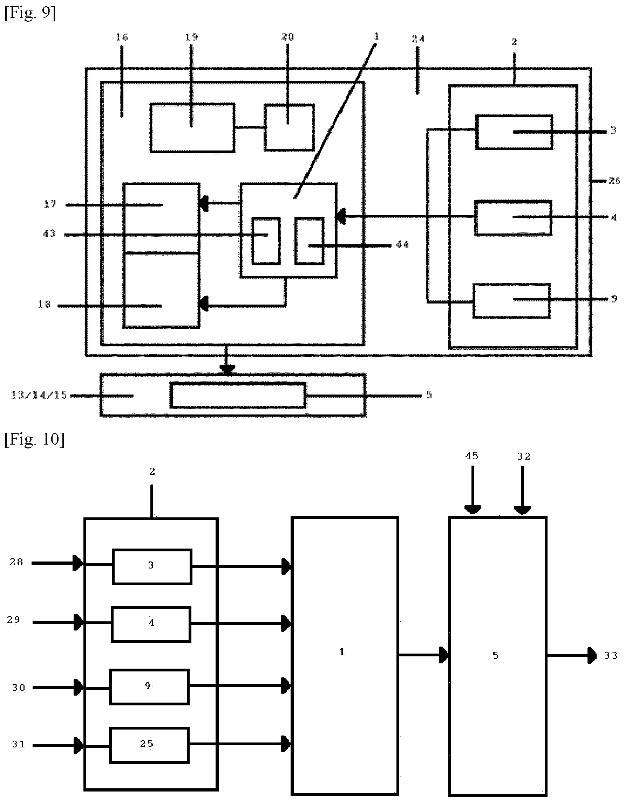

   [fig.9] illustrates a second embodiment of the system according to the present invention incorporated in a patch;
**Fig.10**
   [fig. 10] illustrates a block function diagram of a processing unit and data analysis for cough assessment;
**Fig.11**

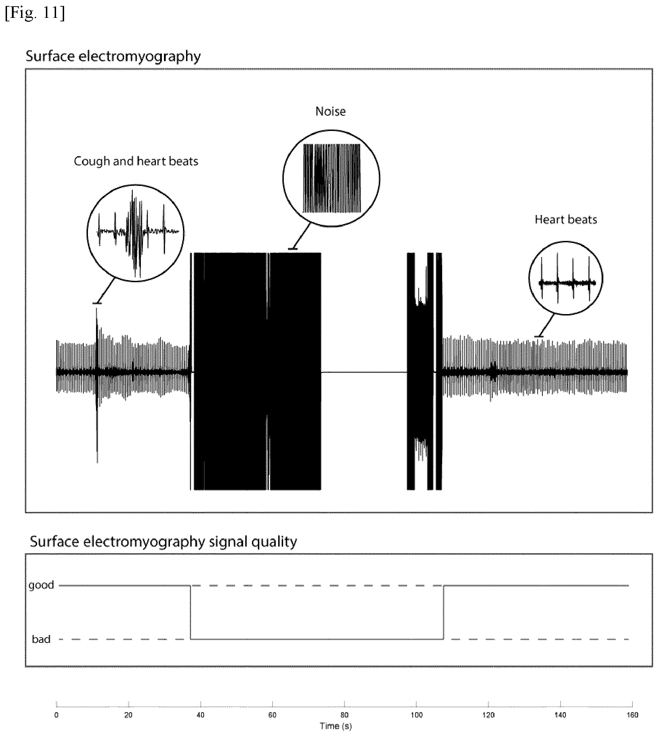

   [fig. 11] illustrates an embodiment of malfunction detection during the collection of sensory signals due to poor contact of the electromyography surface sensor with the user's body;
**Fig.12**

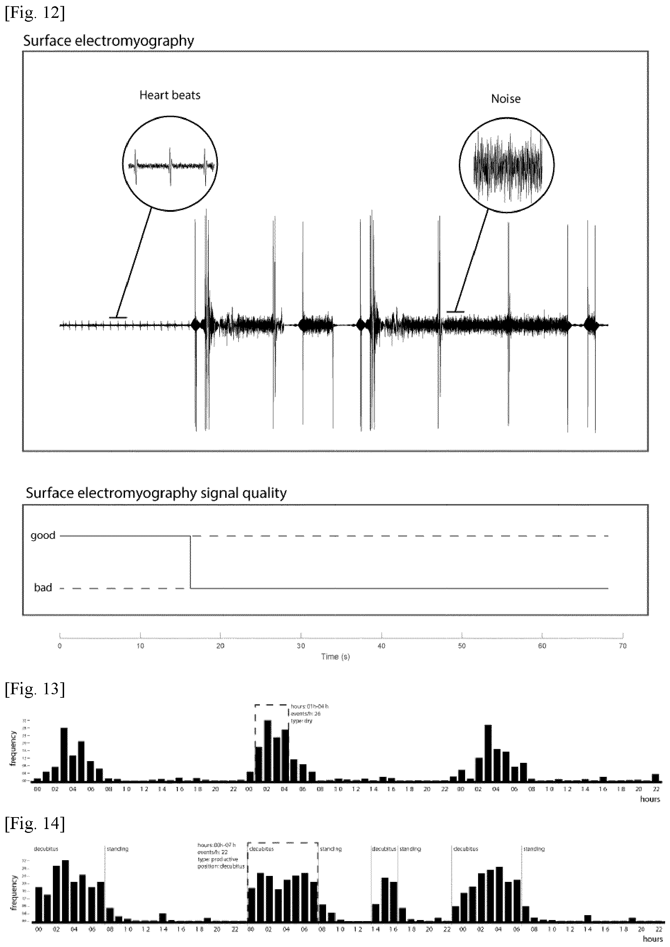

   [fig. 12] illustrates another embodiment of malfunction detection in the collection of sensory signals, due to inadequate contact of the surface electromyography sensor with the user's body;
**Fig.13**
   [fig. 13] illustrates an example of a nocturnal dry cough pattern obtained by the system of the present invention, using an abdominal contraction sensor and an acoustic sensor;
**Fig.14**
   [fig. 14] illustrates an example of a cough pattern obtained by the system of the present invention, using an abdominal contraction sensor, an acoustic sensor and a body positioning and movement characterisation sensor;
**Fig.15**

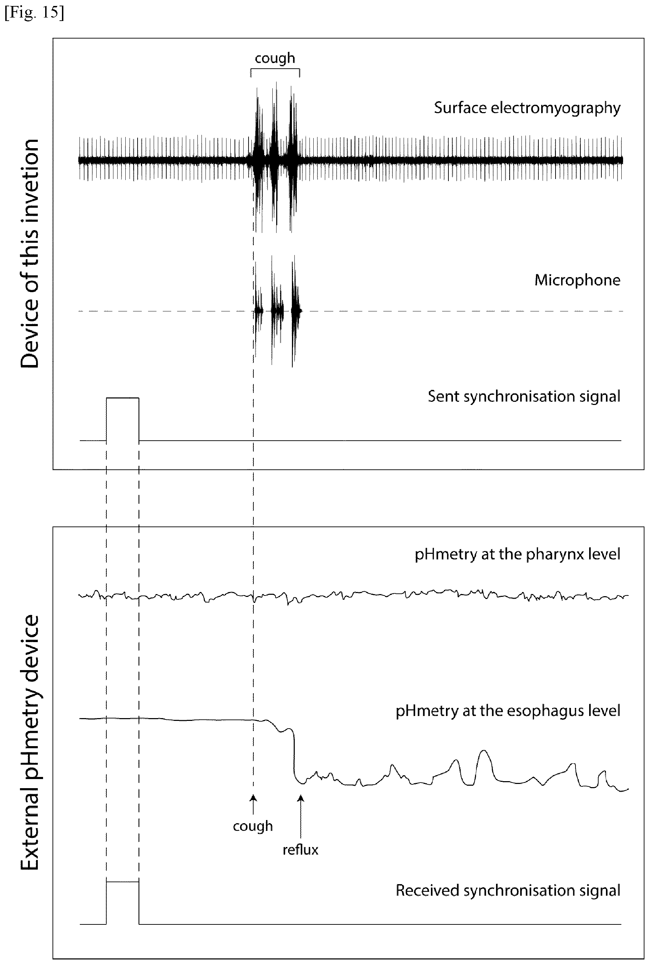

   [fig.15] illustrates an embodiment of the system's synchronization according to the invention with an external pHmetry device, wherein the causal relationship between cough and gastroesophageal reflux is observable.

### Description of Embodiments

The present invention concerns, in a first aspect, a system for monitoring, characterisation and evaluation of a user's cough comprising a sensory instrumentation unit (2), wherein the sensory instrumentation unit (2) includes:
- at least one abdominal contraction sensor (3), adapted to be in contact with the body of a user; and
- at least one acoustic sensor (4), adapted to be in close proximity to the user;

wherein said abdominal contraction sensor (3) and said acoustic sensor (4) collect and transmit raw sensory signals to the sensory instrumentation unit (2);
wherein the sensory instrumentation unit (2) performs the conditioning of said signals of said abdominal contraction sensor (3) and said acoustic sensor (4);
and by comprising a microcontroller (1), wherein the microcontroller (1) includes:
   - a data acquisition unit obtained by the sensory instrumentation unit (43); and
   - an acoustic sensor activation unit (44), which activates said acoustic sensor (4) only when an abdominal contraction obtained from said abdominal contraction sensor (3) is detected, wherein said acoustic sensor (4) only collects and transmits raw sensory signals to the sensory instrumentation unit (2) when it is activated; and
wherein said data acquisition unit obtained by the sensory instrumentation unit (43) and said acoustic sensor activation unit (44) of the microcontroller (1) are interrelated in the sense that the acquisition of data from the acoustic sensor is only carried out when the acoustic sensor activation unit activates it;
and by comprising at least one data flow unit, selected from the group consisting of a data transmission and reception unit (17) and of a data storage unit (18), wherein the data flow unit is controlled by the microcontroller (1);
and by comprising a data processing and analysis unit for cough assessment (5) configured to process and analyse the data resulting from the microcontroller's (1) operation and to monitor, characterise and evaluate the user's cough.

The present invention concerns, in a second aspect, a method for monitoring, characterising and evaluating a user's cough using the system according to the present invention and comprises the following steps:
i. Contact of at least one abdominal contraction sensor (3), comprised in the sensory instrumentation unit (2), with the user's body;
ii. Approach at least one acoustic sensor (4), comprised in the sensory instrumentation unit (2), to close proximity to the user's body;
iii. Transmission of raw sensory signals from the active sensors to sensory instrumentation unit (2);
iv. Conditioning of said signals of said abdominal contraction sensor (3) and of said acoustic sensor (4), if active, by the sensory instrumentation unit (2);
v. Control of the acquisition of data obtained by the sensory instrumentation unit (2) by the microcontroller (1);
vi. Control of the activation of the acoustic sensor (4) by the microcontroller (1) only when an abdominal contraction is detected by said abdominal contraction sensor (3), wherein said acoustic sensor (4) only collects and transmits raw sensory signals to the sensory instrumentation unit (2) when it is activated;
vii. Reception of data from the data processing and analysis unit for cough assessment (5); and
viii. Monitoring, characterisation and evaluation of the user's cough by the data processing and analysis unit for cough assessment (5).

The present invention concerns, in a third aspect, a portable device for monitoring, characterising and evaluating a user's cough which comprises the system according to the present invention and a means of attachment to the user's body.

The present invention concerns, in a fourth aspect, a computer apparatus comprising a processor configured to perform one or more steps of the method according to the present invention.

The present invention concerns, in a fifth aspect, a computer program comprising instructions to enable the computer apparatus according to the present invention to perform one or more steps of the method according to the present invention.

The present invention concerns, in a sixth aspect, a means of reading by a computer apparatus comprising the installation of the computer program according to the present invention.

The system according to the present invention allows, in a surprising way in relation to other known equipment in the state of the art, the provision of reliable and useful parameterized cough patterns for medical diagnosis. The system of the invention makes it possible to create a set of cough patterns based on the frequency and type of cough, obtained through an abdominal contractions and audio sensor. Furthermore, additional cough patterns can be obtained through the incorporation of other sensors and external devices which allow inference about events which can cause cough. Amongst the results provided by the system of the present invention are included: 1) the frequency of cough; 2) the type of cough; and 3) patterns involving the frequency, type of cough and potentially causal cough events. In an embodiment of invention, potentially causal events can be obtained based on their detection by a body positioning and motion characterisation sensor (9). Additionally, gastroesophageal reflux events can be obtained based on a pHmetry device (12). Additionally, abrupt temperature changes can be obtained based on a temperature sensor (11). In addition, abnormal cardiac events can be detected by an electrocardiogram sensor.

Therefore, the invention solves the problem of under-assessment of cough in clinical practice, allowing an objective analysis of a user's cough, contributing to medical diagnosis by establishing parameterized cough patterns, involving the frequency, type of cough, the user's body position and other additional data regarding events which can potentially cause cough.

The system of the present invention can be used for human users of several ages. As will be understood by a person skilled in the art, the system is also applicable to other mammals, including pets and domesticated animals.

The system of the present invention will now be detailed based on the characteristics of each of its main components.

### SENSORY INSTRUMENTATION UNIT

The system of the present invention acquires data from a variety of sensors, namely from at least one abdominal contraction sensor (3), for example a surface electromyography (EMG) sensor with a two-electrode configuration, and at least one acoustic sensor (4). In addition, other sensors can be added, such as a body positioning and motion characterisation sensor (9), a temperature sensor (11), at least one electrocardiogram sensor, among others.

The integration of additional sensors allows the data processing and analysis unit for cough assessment (5) to detect patterns based on potentially causal cough events. For example, the additional sensors allow for the detection of cough patterns which can be attributed to specific underlying diseases. Specifically, the three-axis accelerometer allows the detection of cough causation patterns with the decubitus position, which may indicate that the cough is related to gastroesophageal reflux. On the other hand, the same accelerometer allows for the characterisation of the user's movement, enhancing the correlation of periods of physical activity with the onset of intense cough episodes, which is a characteristic pattern of exercise-induced asthma. Finally, the electrocardiogram sensor allows for the discovery of a pattern of causality between premature ventricular heart beats and cough events.

In the preferred embodiments of the present invention, the sensors used in the system are comprised of a sensory instrumentation unit (2), which can be controlled by a microcontroller (1). The sensory instrumentation unit (2) is responsible for conditioning the signals, specifically the abdominal contraction sensor (3), the acoustic sensor (4) and, when present, the body positioning and motion characterisation sensor (9), the temperature sensor (11) and other additional sensors. As will be detailed throughout the description, the data are conditioned by the sensory instrumentation unit (2) and the control of data acquisition is carried out by the microcontroller (1), which is also responsible for activating the acoustic sensor when abdominal contractions occur. The data processing and analysis unit for cough assessment (5) is responsible for monitoring, characterising and evaluating the user's cough.

The conditioning stage of said active sensor signals, performed by the sensory instrumentation unit (2), is responsible for the initial treatment of the raw sensory signals, namely to attenuate noise and artifacts present in the data acquired by the sensors included in the sensory instrumentation unit (2), employing techniques of conditioning known in the state of the art, as it will be evident to a person skilled in the art.

In the preferred embodiments, the data processing unit for cough assessment (5) uses the data obtained by the microcontroller's operation on the sensors contained in the sensory instrumentation unit, namely the data acquisition and microphone activation, together with data from synchronized external devices and other information such as anthropometric user data.

In the preferred embodiments of the present invention wherein the sensory instrumentation unit (2) also receives raw sensory signals from a body positioning and motion characterisation sensor (9) and performs the conditioning of said signals from this sensor, the following additional steps are employed in the method according to the invention, wherein the data processing and analysis unit for cough assessment (5) evaluates additional cough patterns based on the data from this additional sensor:
i. Contact of the body positioning and motion characterisation sensor (9), comprised in a sensory instrumentation unit (2), with the user's body;
ii. Collection and transmission of sensory signals from the body positioning and motion characterisation sensor (9) to the sensory instrumentation unit (2).

Additionally, the sensory instrumentation unit (2) comprises the connection to one or more external sensory instrumentation units (25), namely additional sensors such as temperature and electrocardiogram sensors.

In a preferred embodiment of the present invention, the sensors used in the sensory instrumentation unit (2) can be either analog or digital. In another embodiment, the additional integration of analog sensors may involve adding one or more external analog-digital converters to the microcontroller (1).

In a preferred embodiment of the present invention, the abdominal contraction sensor (3) is an EMG sensor with a configuration of at least two electrodes. In the even more preferred embodiments, the EMG sensor has a configuration of two electrodes. The EMG sensors are used to monitor the muscular contractions of the user's abdominal wall (epigastric region), which occur during the act of coughing. The EMG sensor, specifically by means of said surface electrodes (6), is placed in specific locations of the skin of the abdominal or epigastric region of the user, in order to optimize the collection of sensory data that allow the capture of the muscular activations resulting from the user's cough.

The sensory instrumentation unit of the EMG sensor uses an instrumentation amplifier to obtain the EMG signal through electrical potentials captured by the surface electrodes. In the sensory instrumentation unit of the EMG sensor an initial noise attenuation is performed through common mode rejection and analog filtering of the signal. Furthermore, signal amplification is performed in order to be easily read by the analog-digital converter of the microcontroller (1).

As illustrated in figure 1, the placing of the EMG surface sensor on the user's body is performed using the electrodes, wherein:
i. an upper electrode of the surface electromyography sensor (7) is contacted with the user's body below the sternum xiphoid process, over the linea alba, i.e. the connective tissue band which corresponds to the midline of the rectus abdominis muscle near the rib cartilages V to VII;
ii. a lower electrode of the surface electromyography sensor (8) is contacted with the user's body on an imaginary line that includes the upper electrode of the surface electromyography sensor (7), parallel to the costal margin, in the epigastric region and over the rectus abdominis muscle. This electrode can be positioned either to the left or right of the midline.
iii. An optional supplementary electrode can be contacted directly over a bone dominant region, namely the iliac crest.

In other embodiments of the present invention, there is the possibility that the additional electrode, which is associated with the EMG sensor, will not be used, which will allow the system to have only one EMG sensor with a configuration of two electrodes connected to the user's body, bringing additional advantages for the invention in relation to the devices of the state of the art which perform cough detection using EMG sensors, in ergonomic terms and of ease of use.

In one of the embodiments of the present invention, the EMG signals are acquired using surface electrodes with gel, which are glued to the skin in the previously mentioned regions. In another embodiment of the present invention, the surface electrodes with gel can be replaced by conductive lycra, which does not contain gel nor is glued to the skin, by gel-free electrodes or other types of electrodes.

An example of sensory signals collected and transmitted by an EMG sensor over a long monitoring period can be seen in figure 2.

As will be evident to a person skilled in the art, each type of abdominal contraction sensor (3) is placed on the user in a different way. Examples of other abdominal contraction sensors are inductance respiratory plethysmography sensors, piezoelectric sensors, or impedance pneumography sensors, which can be placed in different places from those illustrated in figure 1.

In the preferred embodiments of the present invention, the acoustic sensor (4) comprises an omnidirectional microphone, which collects and transmits raw sensory signals to the sensory instrumentation unit (2). The acoustic sensor aims to characterise the type of cough of the user, as well as to support the sensor of abdominal contractions in the detection of cough events. The collection of acoustic sensory signals is controlled by the acoustic sensor activation unit (44), developed to perform the acquisition of data in the instants after the muscular activations or abdominal contractions. This control is performed through the acoustic sensor activation unit (44) of the microcontroller (1), and aims to avoid the recording of private information and superfluous information to characterise the type of cough, which could arise during the continuous monitoring of the voice of the user and those around him. The acoustic sensor can be configured to collect data within 1 to 2 seconds after the detection of abdominal contractions. An example of sensory signals collected and transmitted by an acoustic sensor (4), activated by the detection of abdominal contractions through the signal from the abdominal contraction sensor (3) can be seen in figure 3.

In the preferred embodiments of the present invention, the sensory instrumentation unit (2) comprises at least one body positioning and motion characterisation sensor (9), adapted to collect and send raw sensory signals from the user's body orientation to the sensory instrumentation unit (2). Preferably, the body positioning and motion characterisation sensor (9) is a three-axis accelerometer, adapted to additionally collect and send raw sensory signals from the user's motion to the sensory instrumentation unit (2). The three-axis accelerometer can be used to obtain the patient's body orientation, e.g. lying down (decubitus) or standing up, or other relevant positions, e.g. supine, prone or lateral decubitus position. The body positioning and motion characterisation sensor (9) can also be used to characterise the user's body movements, such as detecting whether the user is standing, running, among other movements. Other examples of body positioning and motion characterisation sensors (9) include a gyroscope or other inertial measurement units.

In the most preferred embodiments of the present invention, the sensory instrumentation unit (2) comprises an abdominal contraction sensor (3), preferably an EMG sensor with a two electrode configuration; an acoustic sensor (4), preferably a microphone; and a body positioning and motion characterisation sensor (9), preferably a three-axis accelerometer.

In other embodiments of the present invention, the sensory instrumentation unit (2) may comprise additional sensors, for example a temperature sensor (11) or an electrocardiogram sensor, wherein each sensor is adapted to collect and send said raw sensory signals to the sensory instrumentation unit (2).

### MICROCONTROLLER

In the preferred embodiments of the invention, the firmware of the system is responsible for giving function to the electronic components and comprises the set of instructions to enable the microcontroller (1) to perform the coordination of sensory data acquisition, data transmission and/or recording, such as the sensory signals coming from each of the sensors, namely the abdominal contraction sensor (3), the acoustic sensor (4), the body positioning and motion characterisation sensor (9), the temperature sensor (11) and the electrocardiogram sensor, among others, and conditioned by the sensory instrumentation unit (2). Furthermore, the firmware is responsible for allowing the integration of additional devices, as detailed below.

The activation step of the acoustic sensor (4) is performed by the acoustic sensor activation unit (44) included in the microcontroller (1), wherein the activation of said sensor occurs only when an abdominal contraction is detected by said abdominal contraction sensor (3), and wherein said acoustic sensor (4) only collects and transmits raw sensorial signals to the sensorial instrumentation unit (2) when it is activated. This technical feature is a solution with unexpected technical effects and not obvious in relation to the systems known in the state of the art, which continuously record audio and, consequently, do not safeguard the user's privacy. According to many users, the previous state of the art devices, which continuously record audio signals, present problems related to the loss of privacy, considering the acquisition of private conversations by these devices, which leads users to be reluctant and uncomfortable to use them.

On the other hand, the activation of the acoustic sensor (4) in the system of the present invention only occurs when abdominal contraction events actually occur, which will also allow a reduction in battery consumption associated with the operation of the system and a reduction in the space required for data storage.

In a preferred embodiment of the invention's system, the acquisition unit of data obtained by the sensory instrumentation unit (43) performs the acquisition of EMG data at a sampling frequency of 1000Hz, audio data at 44100Hz, accelerometer data at 100Hz, temperature sensor at 10Hz and electrocardiogram sensor at 300Hz.

The data from the microphone, which integrates the acoustic sensor (4) will only be acquired and effectively recorded during sensor activations by the acoustic sensor activation unit (44) comprised in the microcontroller (1) only when an abdominal contraction is detected by said abdominal contraction sensor (3). This will be achieved through the firmware of the microcontroller (1) of the system for monitoring, characterisation and evaluation of a user's cough, which will contain a real-time processing block of the abdominal contraction sensor signal, which will detect abdominal contractions and allow the acquisition of audio at set intervals, for example in the following instants, about 1 to 2 seconds, of an abdominal contraction.

As illustrated in figure 4, the acoustic sensor activation unit algorithm (44) comprises the start of sensory signal collection (34) and the acquisition of a sample of a sensory signal by an abdominal contraction sensor (35), wherein the sample of a sensory signal is accumulated by an abdominal contraction sensor into a data buffer of an abdominal contraction sensor (36). It is then checked whether the data buffer has reached a given number of samples, N (37). If it has not, the acquisition of a sample of a sensory signal by an abdominal contraction sensor (35) is resumed. If it has, the algorithm processes the data package (39) in order to check whether it contains an abdominal contraction. This processing includes, in the case of the use of EMG sensors, the application of digital filters in the frequency domain, the Teager Kaiser energy (TKE) operator, and the calculation of the signal's envelope. Other processing techniques can be applied in the case of other abdominal contraction sensors (3). Using the processed data, an algorithm is applied to detect an abdominal contraction event (40), followed by a decision step based on the detection of an abdominal contraction event (41). If an abdominal contraction event has been detected, the activation of the acoustic sensor is performed (42). Regardless of whether an abdominal contraction event has been detected or not, a data buffer cleaning operation is performed (38) with the subsequent resumption of the acquisition of a sample of a sensory signal by an abdominal contraction sensor (35). This process is repeated until the end of the stipulated monitoring period.

The microcontroller (1) acquires the conditioned signals from the active sensors of the sensory instrumentation unit (2), which are transmitted to the data processing and analysis unit for cough assessment (5).

The microcontroller (1) can be configured to control the electronics blocks of the system illustrated in figure 6. That is, it can be configured to directly control the sensory instrumentation unit (2), the data flow unit, which is selected from the group consisting of a data transmission and reception unit (17) and a data storage unit (18). In one of the preferred embodiments of implementation of the present invention a low power consumption microcontroller (1) with Advanced RISC Machine (ARM) architecture is used.

In an embodiment of the invention, the microcontroller (1) can also receive commands sent by the data processing and analysis unit for cough assessment (5) through the data transmission and reception unit (17), wherein the commands may relate, for example, to instructions to start or end a sensory data acquisition period.

### CENTRAL MODULE AND PORTABLE DEVICE

According to the different embodiments of the present invention, as will be evident to a person skilled in the art, the central module (16) of the system for monitoring, characterising and evaluating a user's cough may comprise any data transmission and reception unit (17). The data transmission and reception unit (17) may be an electronic unit adapted to transmit the data to the data processing and analysis unit for cough assessment (5), wherein said electronics is, for example, a Bluetooth module. The digital sensory signals can be transmitted, for example, via Bluetooth or Low Energy Bluetooth or other wireless data transmission technology, such as using radio frequency range waves or via Wi-Fi, to a smartphone, a computer, a tablet, a smartwatch or for any other computer apparatus (13), wherein said computer apparatus (13) comprises the data processing and analysis unit for cough assessment (5).

In the embodiments of the present invention wherein the system comprises a data storage unit (18), this may be a memory card, for example an SD card. The data stored in the memory card may be downloaded into a computer apparatus (13) comprising the data processing and analysis unit for cough assessment (5) or they may be downloaded into a server (14) or cloud computing (15) comprising the data processing and analysis unit for cough assessment (5). Thus, the digital sensory signals stored in the memory card may be processed locally, i.e. into a smartphone, on a local computer or tablet, and/or transmitted to cloud computing (cloud) or server and remotely undergo processing in the network.

Both data transmission and data storage options can occur simultaneously, for example simultaneous recording to an SD card and transmission of the data to the data processing and analysis unit for cough assessment (5).

In other preferred embodiments according to the invention, a data processing and analysis unit for cough assessment (5) is included in the microcontroller (1) itself, wherein the system according to the present invention comprises a central module (16) including the microcontroller (1) and:
- at least one energy source (19); and
- at least one data transmission and reception unit (17), adapted to transmit the data resulting from the microcontroller's (1) operation, wherein the microcontroller (1) controls the data transmitted by the data transmission and reception unit (17), wherein the data is transmitted via wired communication (21) or a wireless communication protocol (22);
and the microcontroller (1) comprises the data processing and analysis unit for cough assessment (5).

In another embodiment wherein the data processing and analysis unit for cough assessment (5) is included in the microcontroller (1) itself, the system according to the present invention comprises a central module (16) including the microcontroller (1) and:
- at least one energy source (19); and
- at least one data storage unit (18), adapted to store the data resulting from the microcontroller's (1) operation, wherein the microcontroller (1) controls the data stored by the data storage unit (18);
and that the microcontroller (1) comprises the data processing and analysis unit for cough assessment (5).

As illustrated in Figure 7, in another embodiment wherein the data processing and analysis unit for cough assessment (5) is included in the microcontroller (1) itself, the system according to the present invention comprises a central module (16) including the microcontroller (1) and:
- at least one energy source (19); and
- at least one data transmission and reception unit (17), adapted to transmit the data resulting from the microcontroller's (1) operation, wherein the microcontroller (1) controls the data transmitted by the data transmission and reception unit (17), wherein the data is transmitted via wired communication (21) or a wireless communication protocol (22); and
- at least one data storage unit (18), adapted to store the data resulting from the microcontroller's (1) operation, wherein the microcontroller (1) controls the data stored by the data storage unit (18);
and that the microcontroller (1) comprises a data processing and analysis unit for cough assessment (5).

In the embodiment illustrated in figure 7, the system is not linked to the operation of a computer apparatus (13), a server (14) or a cloud computing (15), because the data processing and analysis unit for cough assessment (5) is incorporated in the central module (16), more precisely in the microcontroller (1).

The embodiment illustrated in Figure 7 is particularly advantageous when the system according to the present invention is incorporated into a portable device for monitoring, characterisation and evaluation of a user's cough, wherein said device includes a means for attachment to the user's body.

The means of attachment known in the state of the art, which can be employed on the devices of the present invention, are, for example, a grasping clip which can be fitted/fixed to the waistband of the trousers or of a pocket; or by means of a grasping belt, which is placed around the waist of the user.

However, as will be evident to a person skilled in the art, the portable device according to the present invention also comprises the system embodiment illustrated in Figure 6, wherein the central module (16) comprises a microcontroller (1), adapted to receive the sensory signals conditioned by the sensory instrumentation unit (2) and wherein the data processing and analysis unit for cough assessment (5) is comprised in a computer apparatus (13), a server (14) or cloud computing (15).

In other embodiments of the present invention, the central module (16) may comprise a screen (23), for example a touch screen, adapted to display information and/or to enter commands, such as pauses in monitoring, personal information of the user, anthropometric information of the user, and mark events, such as a symptom experienced by the user or the administration of drugs, for the operation of the data processing and analysis unit for cough assessment (5).

In other embodiments of the present invention, the central module (16) may comprise one or more push buttons to mark events, such as a symptom experienced by the user or the administration of drugs, for the operation of the data processing and analysis unit for cough assessment (5).

In the embodiments of the device according to the present invention, which use the systems illustrated in Figures 6 and 7, the sensors, namely the abdominal contraction sensor (3), the acoustic sensor (4) and the body positioning and motion characterisation sensor (9), derive from a casing, e.g. a box, which encloses the portable device, wherein the sensors are connected to the user either through a wired communication or a combination of wireless communication protocols.

In other preferential embodiments of the present invention, as illustrated in Figures 8 and 9, the casing comprises a miniaturized, portable device in the form of an adhesive patch, also known as a patch (24), i.e. it sticks to the user's body as if it were an adhesive bandage, with the integrated sensory instrumentation (2), namely the abdominal contraction sensor (3), for example an EMG sensor, the acoustic sensor (4) and, optionally, the body positioning and motion characterisation sensor (9), for example a three-axis accelerometer, in addition to other additional sensors. The acoustic sensor (4) can be placed in an area of the portable device that does not come into contact with the user's skin, in order to enhance the reception of acoustic signals. Alternatively, the acoustic sensor (4) can be decoupled from the patch and positioned outside of it, wherein acoustic signals are collected only when abdominal contraction events occur, and transmitted to the central module (16) through one or more wires or a combination of wireless communication protocols.

The components of the central module (16) illustrated in Figures 6 to 9 are described in more detail below.

The energy source (19), for example one or more batteries, supplies all the units of the central module (16) of the system for monitoring, characterisation and evaluation of a user's cough. Preferably the energy source will allow an extended monitoring period of at least 24 hours. The batteries which can be used in the system can be lithium cell batteries. The charging of the batteries can be done by methods commonly known in the state of the art, including by wire or inductive charging. As will be evident to a person skilled in the art, the batteries known in the state of the art are able to be used in the central module (16) of the present invention, in the same way as other energy sources, such as those which include the collection of thermoelectric energy, enhanced by the contact of the casing (patch) to the user's body.

In the preferred embodiments, the central module (16) has a voltage and temperature regulator (20) connected to the power source (19) rechargeable, wherein the voltage regulator circuit is responsible for keeping the circuit voltage at 3.3V, even when the voltage of the power source (19) is above this value. When the voltage drops below 3.3V, the central module (16) is automatically safely switched off by the microcontroller (1), as controlled by firmware.

The voltage and temperature regulator (20) of the batteries or cells ensures the safety conditions of the system for monitoring, characterisation and evaluation of a user's cough. Batteries or cells, for example those composed of lithium cells, can be dangerous if exposed to extreme conditions. In particular, temperature fluctuations are one of the main reasons for the occurrence of data in batteries. Therefore, the system for monitoring, characterisation and evaluation of a user's cough includes a voltage and temperature regulator (20) to prevent problems related to it. Finally, the charging circuit will allow users to charge the power source (19) of the central module (16) of the system for monitoring, characterisation and evaluation of a user's cough. This circuit is responsible for maintaining both the potential and the constant current for appropriate battery charging.

In the embodiments wherein the sensory instrumentation unit (2) is not coupled to the central module (16) and in the embodiments wherein some of the components of the sensory instrumentation unit (2), such as the acoustic sensor (4), are also not coupled to the central module (16), the communication with the central module (16) will occur through a wireless communication protocol (22), which will mean the uncoupled components have their own energy source. Additionally, the uncoupled components comprise adapted electronics to communicate with the system of the present invention.

In the preferred embodiments of the invention, users perform the monitoring and, during that time, the digital sensory signals are transmitted, through a wireless communication protocol (22), for the data processing and analysis unit for cough assessment (5) to perform the method steps according to the present invention on the network, for example on a server (14) or in cloud computing (16).

Therefore, in the embodiments of the present invention it is possible to use the systems of the present invention to perform the monitoring, characterisation and evaluation of a user's cough by telemedicine, wherein data and cough patterns are transmitted through wireless communication protocols to remote locations.

The systems according to the present invention can also include a computer clock, which would be useful for scheduling monitoring sessions, such as the beginning and/or end of sensory data acquisition.

Systems according to the present invention may also comprise a decoupling subsystem, as will be evident to a person skilled in the art in view of safety matters in case the user is monitoring himself with the device connected to the current.

The systems according to the present invention can also be inserted in a water-resistant unit, which is especially advantageous to allow users to really use the device in its full ergonomic way, being able to live their life as usual, without interrupting the data collection during a bath for example.

### SYNCHRONIZATION WITH EXTERNAL DEVICES

In addition to additional sensor integration, the present invention also features means of synchronization with external devices to enable the data processing and analysis unit for cough assessment (5) to detect patterns based on potentially causal cough events. Similar to the integration of additional sensors, synchronization with external devices provides a more complete framework for generating patterns associated with user cough events, e.g. causality patterns. In even more preferred embodiments, the data processing and analysis unit for cough assessment (5) monitors, characterises and evaluates additional cough patterns based on data obtained through synchronization with other external devices.

In the preferred embodiments of the invention, the system can be synchronized with a pHmetry device (12). Specifically, the synchronization of the present invention with a pHmetry device allows a possible causal pattern to be established between coughing and gastroesophageal reflux, in order to understand whether, in the first instance, it is the coughing events which cause the reflux or whether it is the reflux episodes which cause the cough.

In the preferred embodiments of the invention, the microcontroller (1) is responsible for synchronization with other external devices. In one embodiment, the synchronization can be performed through a synchronization signal, wherein the synchronization signal can be transmitted to the external device through a wired communication (21) or through a wireless communication protocol (22). In this embodiment, the same synchronization signal is transmitted to the data processing and analysis unit for cough assessment (5) through the data flow unit. In the same embodiment, the external device works in parallel and independently, and the sensory signals of both devices are then temporally aligned in the data processing and analysis unit for cough assessment (5) through the temporal alignment of the synchronization signal.

In another embodiment, it is possible to synchronise the invention system with external devices by synchronising the internal clocks of the devices' microcontrollers or data transmission and reception units (namely a Bluetooth module).

Alternatively, in another embodiment, the synchronization between devices can be performed using a computer program. In one embodiment, the computer program is responsible for sending a synchronization event to both devices, allowing the synchronization to be performed later on the data processing and analysis unit for cough assessment (5) based on that synchronization event.

As illustrated in figure 15, the system of the present invention can be synchronized with an external device, for example a pHmetry device (12). In the illustrated embodiment, the device of the present invention captures the signals of abdominal contractions, namely through an EMG sensor, and captures the audio signals after activation by the acoustic sensor activation unit, included in the microcontroller (1), namely through an omnidirectional microphone. In parallel, an external pHmetry device captures pHmetry signals at the pharyngeal and esophagus levels. As illustrated in figure 15, the device of the present invention synchronizes with said external device by sending a synchronization signal, for example a step function, which is received by the external device. This synchronization signal has the function of establishing an alignment time frame for the joint analysis of the sensory data from the different devices involved in the process of monitoring, characterisation and evaluation of a user's cough. Subsequently, the data processing and analysis unit for cough assessment (5) performs the temporal alignment of the digital sensory signals from both devices based on the temporal alignment of the synchronization signal. After the alignment of the signals from both devices, the data processing and analysis unit for cough assessment (5) processes and analyses the set of sensory signals from both devices and establishes a causality pattern between the cough and the associated gastroesophageal reflux episode.

### MALFUNCTION UNIT OF THE SYSTEM

As illustrated in figures 11 and 12, the system according to the present invention can present malfunction detection functionalities of the system itself by means of a sensory instrumentation unit malfunction detection unit (27). The data in figures 11 and 12 show an anomaly in the data acquisition of the abdominal contraction sensor (3), wherein the detection of the anomaly can be performed in the firmware of the microcontroller, the code being adapted, to detect whether the signals have values or characteristics that are associated with the malfunction of the system. Specifically, figure 11 is an example wherein the EMG sensor is no longer in contact with the skin of the user. Specifically, figure 12 is an example wherein the EMG sensor, particularly the electrodes, are not correctly in contact with the skin of the user due to its high pilosity.

Another embodiment of the malfunction detection unit may include a digital output of the EMG sensor which indicates whether the sensor is correctly in contact with the skin.

### PROCESSING AND ANALYSIS OF COUGH ASSESSMENT DATA

In a preferred embodiment according to the invention, the sensory signals obtained by the sensory instrumentation unit (2) and the external devices are processed by a computer program run by the data processing and analysis unit for cough assessment (5) to continue the monitoring, characterisation and evaluation of a user's cough. In particular, the invention system allows for the objective analysis of a user's cough by establishing parameterized cough patterns involving frequency, type of cough, user's body position and other supplementary data on potentially causal cough events. This computer program uses sensory signal processing techniques in addition to various automatic learning algorithms (machine learning) and artificial intelligence, to analyse, process and classify digital sensory data.

The data processed and analysed by the data processing and analysis unit for cough assessment (5) comprises the following data and its combinations:
- digital sensory signals obtained from the sensory instrumentation unit (2), including signals obtained from an external sensory instrumentation unit (25);
- digital sensory signals obtained from external devices;
- digital sensory signals resulting from the microcontroller (1);
- digital sensory signals transmitted by the data transmission and reception unit (17);
- digital sensory signals stored on the data storage unit (18);
- anthropometric data, in particular entered via the screen (23);
- personal data, e.g. variables related to gender and age, namely entered using the screen (23);
- other events recorded by the user, for example a symptom experienced by the user; and
- synchronization inputs, for example synchronization signals.

The EMG sensors, through the action of the data processing and analysis unit for cough assessment (5), allow the detection of cough events, and it is also possible to use a joint action with the acoustic sensor (4), for example a microphone, to improve the performance of the cough detection algorithm, namely to decrease the false positive rate. However, in other embodiments of the present invention, the detection of abdominal contraction events may not be dependent on the audio signal, i.e., the detection of cough events can be done only through the processing of the abdominal contraction sensor signals (3) by the data processing and analysis unit for cough assessment (5).

The acoustic signal, through the action of the data processing and analysis unit for cough assessment (5), allows the characterisation of the type of cough. The signals of abdominal contractions events can also be used to characterise the type of cough. In one embodiment of the present invention, the cough can be classified as dry cough and productive cough. In another embodiment, the data processing and analysis unit for cough assessment (5) can also detect other additional characteristics of cough, such as whether the cough is accompanied by wheezing.

Regarding the types of cough patterns which can be established by the present invention, as illustrated in Figure 13, the system of the present invention can detect a cough pattern, for example a dry nocturnal cough pattern based on the use of an EMG sensor (3) and an acoustic sensor (4).

User anthropometric information such as weight, height and age can also be used by the data processing and analysis unit for cough assessment (5) for cough detection and characterisation.

In the preferred embodiments of the invention, the data processing and analysis unit for cough assessment (5) evaluates additional cough patterns based on data from a supplementary body positioning and motion characterisation sensor (9). Take as an example the pattern illustrated in figure 14, wherein a pattern was detected indicating that the decubitus (lying down) position triggers cough episodes.

In the even more preferred embodiments, the data processing and analysis unit for cough assessment (5) monitors, characterises and evaluates additional cough patterns based on data from additional sensors and external devices, which provides data on potentially causal cough events. In a preferred embodiment, the data processing and analysis unit for cough assessment (5) allows temporal alignment of data obtained through the sensory instrumentation unit (2) with data from external devices, as previously described in more detail.

The sensory signals collected by the EMG sensor, positioned in the user's abdominal region, comprise artifacts that correspond to signals derived from the heartbeat. The data processing and analysis unit for cough assessment (5) can be configured to detect cough events by filtering and discarding the signals derived from the heartbeat. However, the filtered and discarded heartbeat signals can be displayed and processed. Thus, as illustrated in Figure 5, the system according to the present invention also allows for the monitoring of the user's heart rate. On the other hand, for the detection of heartbeats with altered morphology, such as premature ventricular beats, an additional electrocardiogram sensor can be incorporated in other embodiments of the invention. The signals from this sensor can also be processed by the data processing and analysis unit for cough assessment (5) to generate additional cough patterns associated with cardiovascular problems.

In one of the embodiments of the present invention, the automatic learning algorithms can detect cough patterns using data regarding the frequency and type of cough, such as night cough patterns, as illustrated in figure 13, and informative data on potentially causal cough events, such as the cough triggered by decubitus position, as illustrated in figure 14, the cough triggered by physical exercise, or the cough triggered by sudden changes in temperature.

In one of the embodiments of the present invention the types of algorithms and the machine learning features, regarding the system for monitoring, characterisation and evaluation of a user's cough, the automatic learning classifiers can range from simple decision trees and k-nearest neighbours classifiers, to complex neural networks and deep learning algorithms.

Preferably, the automatic learning algorithms, used by the data processing and analysis unit for cough assessment (5), combine the relevant characteristics extracted from the signals obtained by the sensory instrumentation unit (2) and external devices, or possibly a set of raw sensory signals, to allow quantitative and qualitative cough characterisation.

User anthropometric information such as weight, height and age may also be useful in detecting cough events and the type of cough.

In an embodiment of the invention, data from the event marking, namely using a screen installed in the invention system, can also be used to detect cough patterns. Specifically, a pattern can be detected wherein the user feels palpitations in the moments before cough episodes, which may indicate that the cough is associated with cardiovascular problems.

In the preferred embodiments of the present invention, the data processing and analysis unit for cough assessment (5) comprises instructions read by a computer which can be executed on a computer apparatus (13), a server (14), cloud computing (15) or a microcontroller (1).

The computer program contained in the data processing and analysis unit for cough assessment (5) may be executed locally, by running it directly on a computer apparatus (13). In one of the preferred embodiments of the present invention, a computer apparatus (13) comprises the data processing and analysis unit for cough assessment (5), and said computer apparatus (13) may be a computer, such as a local computer, a smartphone, a smartwatch or a tablet. Alternatively, such a computer program can be run on the network. In this embodiment the digital sensory data is transmitted to a server (14) or a cloud computing (15) and processed there. Therefore, a server (14) or a cloud computing (15) can comprise the data processing and analysis unit for cough assessment (5).

In the preferred embodiments wherein the data processing and analysis unit for cough assessment (5) is comprised in a computer apparatus (13), a server (14) or cloud computing (15), the system according to the present invention comprises a central module (16) including the microcontroller (1) and:
- at least one energy source (19); and
- at least one data transmission and reception unit (17), adapted to transmit the data resulting from the microcontroller's (1) operation, wherein the microcontroller (1) controls the transmission of the data by the data transmission and reception unit (17), wherein the data is transmitted via wired communication (21) or via a wireless communication protocol (22);
and wherein the data processing and analysis unit for cough assessment (5) processes and analyses the data transmitted by the data transmission and reception unit (17).

Alternatively, in the preferred embodiments wherein the data processing and analysis unit for cough assessment (5) is comprised of a computer apparatus (13), a server (14) or cloud computing (15), the system according to the present invention comprises a central module (16) including the microcontroller (1) and:
- at least one energy source (19); and
- at least one data storage unit (18), adapted to store the data resulting from the microcontroller's (1) operation, wherein the microcontroller (1) controls the storage of the data by the data storage unit (18);
and for the data processing and analysis unit for cough assessment (5) to process and analyse the data stored in the data storage unit (18).

Alternatively, as illustrated in Figure 6, in the preferred embodiments wherein the data processing and analysis unit for cough assessment (5) is comprised of a computer apparatus (13), a server (14) or cloud computing (15), the system according to the present invention comprises a central module (16) including the microcontroller (1) and:
- at least one energy source (19); and
- at least one data transmission and reception unit (17), adapted to transmit the data resulting from the microcontroller's (1) operation, wherein the microcontroller (1) controls the data transmitted by the data transmission and reception unit (17), wherein the data is transmitted via wired communication (21) or via a wireless communication protocol (22); and
- at least one data storage unit (18), adapted to store the data resulting from the microcontroller's (1) operation, wherein the microcontroller (1) controls the data stored by the data storage unit (18);
and wherein the data processing and analysis unit for cough assessment (5) processes and analyses the data transmitted by the data transmission and reception unit (17) and the data stored in the data storage unit (18).

In an embodiment of the invention, the data processing and analysis unit for cough assessment (5) can send commands to the data transmission and reception unit (17), wherein the commands may relate, for example, to instructions to start or end a sensory data acquisition period.

As illustrated in Figure 10, a block function diagram of the invention comprises the reception of abdominal contraction data (28) by an abdominal contraction sensor (3), e.g., an EMG sensor; the reception of audio signals only during the instants following abdominal contraction events (29) by an acoustic sensor (4) comprising a microphone, e.g., the reception of input data for user body position and motion (30) by a body positioning and motion characterisation sensor (9), e.g., a three-axis accelerometer; and the reception of a data set from an external sensor (31) by an external sensory instrumentation unit (25). During acquisition, the microcontroller (1) controls the activation of the acoustic sensor (4), via the acoustic sensor activation unit (44), when an abdominal contraction event is detected by the abdominal contraction sensor (3). The digital sensory data obtained by the microcontroller (1) is sent to a data processing and analysis unit for cough assessment (5), which comprises a signal processing block and may also comprise an automatic machine learning classification block. In addition to the sensory data obtained by the sensory instrumentation unit (2) and external instrumentation units, the data processing and analysis unit for cough assessment (5) may also receive at least one anthropometric input information (32) and at least one set of data from a synchronized external device (45). At the end of processing, the data processing and analysis unit for cough assessment (5) presents as a result, based on the stipulated cough monitoring period, a set of output information (33): the frequency and type of cough events; and the parameterized cough patterns, involving the frequency, type of cough, body position and movements of the user and other supplementary data on potentially causal cough events.

The signals from the abdominal contraction sensor will, in a first step, be conditioned by the data processing and analysis unit for cough assessment (5) in order to remove artifacts, namely network noise (50Hz or 60Hz) and other artifacts. The processing, conducted by the data processing and analysis unit for cough assessment (5), comprises the use of digital filters in the frequency domain, for example low-pass, band-pass and band rejection filters. Furthermore, the data processing and analysis unit for cough assessment (5) removes or attenuates the heartbeats which are present in the signal from the electromyography sensor. This decontamination of the signal can be performed through various signal processing algorithms, such as filters in the frequency domain and dynamic filtering using the Savitzky-Golay filter. Finally, after this step, the Teager Kaiser operator is applied in order to increase the signal-to-noise ratio between the muscle activations and the baseline.

The processing performed by the data processing and analysis unit for cough assessment (5) also comprises the abdominal contraction event identification step, which aims to detect all possible abdominal contractions, whether they are caused by coughing events or not, since the automatic learning step will later differentiate the activations which actually correspond to the cough. In this way, this step will be optimized so that all abdominal activations are detected, in order to ensure that no coughing events are lost. Additionally, for the EMG sensor, the processing performed by the data processing and analysis unit for cough assessment (5) may comprise a signal filtering step with a moving average filter in order to smooth the signal and facilitate the detection of abdominal contraction event activations; and a stage of detection and delineation of the activations, i.e., detection of the beginning and end of the activations, through a dynamic threshold, which will be adjusted according to the signal snippet to be analysed.

After the abdominal contractions are identified and delineated, their signal will be used by the data processing and analysis unit for cough assessment (5) to extract characteristics, which will be used by the automatic learning algorithms. The extracted characteristics include: activation duration, area and maximum amplitude, among other characteristics in the time, frequency and time-frequency domain (wavelets).

The audio signal which is processed by the data processing and analysis unit for cough assessment (5), which runs the computer program adapted to perform the method's steps according to the present invention, corresponds to audio excerpts recorded as a consequence of activations by the acoustic sensor activation unit (44), with the purpose of obtaining the audio only in the moments after the abdominal contractions. In the preferred embodiments of the present invention, the data processing and analysis unit for cough assessment (5) contains a pre-processing step of said audio excerpts, with the purpose of removing artifacts. Afterwards, the signal snippets can be used to extract characteristics, similarly to what is done for the signal emitted by the abdominal contraction sensor (3), which will be used by the automatic learning algorithms. Alternatively, the signal snippet can be directly used by the automatic learning algorithms.

In the preferred embodiments of the invention, additional sensors can be coupled to the sensory instrumentation unit. The external devices, in turn, have to be synchronized to the invention system, given its independent operation. Additionally, the external devices can send data to the data processing and analysis unit for cough assessment (5).

In another embodiment of the invention, wherein the data processing and analysis unit for cough assessment (5) receives the additional sensory signals from additional sensors or additional external devices, that unit is also responsible for processing said data from the external sensors or devices and eventually detecting patterns based on that data. These additional patterns on potentially causal cough events provide a more complete picture of the generation of patterns associated with user cough events.

As used in this description, the expression "being in contact with a user's body" or the expression "contact with a user's body" refers to the positioning of a sensor or device on a user's skin, namely on an abdominal or epigastric region.

As used in this description, the expression "being in close proximity to the user" or the expression "approaching the body of a user" refers to the positioning of a sensor or device at a certain distance from the user that allows effective operation of that sensor or device.

As used in this description, the terms "about" and "approximately" refer to a value range of plus or minus 10% of the specified number.

As used in this description, the expression "substantially" means that the actual value is within about 10% of the desired value, variable or related limit, particularly within about 5% of the desired value, variable or related limit or especially within about 1% of the desired value, variable or related limit.

The subject matter described above is provided as an illustration of the present invention and should not be interpreted in such a way as to limit it. The terminology used for the purpose of describing specific embodiments in accordance with the present invention should not be interpreted to limit the invention. As used in the description, the defined and undefined articles, in their singular form, aim at the interpretation of including plural forms as well, unless the context of the description explicitly indicates otherwise. It will be understood that the terms "comprise" and "include", when used in this description, specify the presence of the characteristics, elements, components, stages and related operations, but do not exclude the possibility that other characteristics, elements, components, stages and operations are also included.

All changes, as long as they do not modify the essential characteristics of the following claims, shall be considered within the scope of protection of the present invention.

### Reference Signs List

- 1. -: a microcontroller;
- 2. -: a sensory instrumentation unit;
- 3. -: an abdominal contraction sensor;
- 4. -: an acoustic sensor;
- 5. -: a data processing and analysis unit for cough assessment;
- 6. -: a surface electrode;
- 7. -: a top electrode from the surface electromyography sensor;
- 8. -: a lower electrode of the surface electromyography sensor;
- 9. -: a body positioning and motion characterisation sensor;
- 10. -: a superior surface electromyography (EMG) sensor;
- 11. -: a temperature sensor;
- 12. -: a pHmetry device;
- 13. -: a computer apparatus;
- 14. -: a server;
- 15. -: a cloud computing;
- 16. -: a central module;
- 17. -: a data transmission and reception unit;
- 18. -: a data storage unit;
- 19. -: an energy source;
- 20. -: a voltage and temperature regulator;
- 21. -: a wired communication;
- 22. -: a wireless communication protocol;
- 23. -: a screen;
- 24. -: a patch;
- 25. -: an external sensory instrumentation unit;
- 26. -: a water-resistant unit;
- 27. -: a sensory instrumentation unit's malfunction detection unit;
- 28. -: a reception of abdominal contraction data;
- 29. -: an audio signal reception only in the moments after abdominal contraction events;
- 30. -: a reception of input data of the user's body position and movements;
- 31. -: an external sensor data set;
- 32. -: anthropometric input information;
- 33. -: a set of output information;
- 34. -: a start of sensory signal collection;
- 35. -: an acquisition of a sample of a sensory signal by an abdominal contraction sensor;
- 36. -: an accumulation of the sample of a sensory signal by an abdominal contraction sensor to a data buffer of an abdominal contraction sensor;
- 37. -: a verification that the data package has reached a given number of samples;
- 38. -: a data buffer cleaning operation;
- 39. -: a data buffer processing;
- 40. -: an application of an algorithm to detect an abdominal contraction event;
- 41. -: a decision based on the detection of an abdominal contraction event;
- 42. -: an acoustic sensor activation;
- 43. -: a data acquisition unit obtained by the sensory instrumentation unit;
- 44. -: an acoustic sensor activation unit (44); and
- 45. -: a data set from an external synchronized device (45).

## Claims

1. A system for monitoring, characterisation and evaluation of a user's cough, **characterised by** comprising a sensory instrumentation unit (2), wherein the sensory instrumentation unit (2) includes:
• at least one abdominal contraction sensor (3), adapted to be in contact with the body of a user; and
• at least one acoustic sensor (4), adapted to be in close proximity to the user;
wherein said abdominal contraction sensor (3) and said acoustic sensor (4) collect and transmit raw sensory signals to the sensory instrumentation unit (2);
wherein the sensory instrumentation unit (2) performs the conditioning of said signals of said abdominal contraction sensor (3) and said acoustic sensor (4);
and by comprising a microcontroller (1), wherein the microcontroller (1) includes:
• a data acquisition unit obtained by the sensory instrumentation unit (43); and
• an acoustic sensor activation unit (44), which activates said acoustic sensor (4) only when an abdominal contraction on the signal obtained from said abdominal contraction sensor (3) is detected, wherein said acoustic sensor (4) only collects and transmits raw sensory signals to the sensory instrumentation unit (2) when it is activated;
wherein said acquisition unit of the data obtained by the sensory instrumentation unit (43) and said acoustic sensor activation unit (44) of the microcontroller (1) are interrelated;
and by comprising at least one data flow unit, selected from the group consisting of a data transmission and reception unit (17) and of a data storage unit (18), wherein the data flow unit is controlled by the microcontroller (1);
and by comprising a data processing and analysis unit for cough assessment (5) configured to process and analyse the data resulting from the microcontroller's action (1) and to monitor, characterise and evaluate the user's cough.

2. The system for monitoring, characterisation and evaluation of a user's cough, according to the previous claim, **characterised by** the abdominal contraction sensor (3) is a surface electromyography (EMG) sensor, with a configuration of at least 2 electrodes.

3. The system for monitoring, characterisation and evaluation of a user's cough, according to any of the previous claims, **characterised by** the sensory instrumentation unit (2) comprises at least one body positioning and motion characterisation sensor (9), adapted to collect and send raw sensory signals to the sensory instrumentation unit (2).

4. The system for monitoring, characterisation and evaluation of a user's cough, according to the previous claim, **characterised by** the body positioning and motion characterisation sensor (9) is a three-axis accelerometer.

5. The system for monitoring, characterisation and evaluation of a user's cough, according to any of the previous claims, **characterised by** the sensory instrumentation unit (2) comprises the connection to one or more external sensory instrumentation units (25).

6. The system for monitoring, characterisation and evaluation of a user's cough, according to any of the previous claims, **characterised by** comprising means adapted for synchronization with other devices.

7. The system for monitoring, characterisation and evaluation of a user's cough, according to any of the previous claims, **characterised by** a computational apparatus (13) comprising the data processing and analysis unit for cough assessment (5).

8. The system for monitoring, characterisation and evaluation of a user's cough, according to any of the claims 1 to 6, **characterised by** a server (14) or cloud computing (15) comprising the data processing and analysis unit for cough assessment (5).

9. The system for monitoring, characterisation and evaluation of a user's cough, according to any of the claims 7 and 8, **characterised by** comprising a central module (16) including the microcontroller (1) and
- at least one energy source (19); and
- at least one data transmission and reception unit (17), adapted to transmit the data resulting from the microcontroller's (1) operation, wherein the microcontroller (1) controls the data transmission by the data transmission and reception unit (17), wherein the data is transmitted via a wired communication (21) or via a wireless communication protocol (22);
and by the data processing and analysis unit for cough assessment (5) process and analyse the data transmitted by the data transmission and reception unit (17).

10. The system for monitoring, characterisation and evaluation of a user's cough, according to any of the claims 7 and 8, **characterised by** comprising a central module (16) including the microcontroller (1) and
- at least one energy source (19); and
- at least one data storage unit (18), adapted to store the data resulting from the microcontroller's (1) operation, wherein the microcontroller (1) controls the storage of the data by the data storage unit (18);
and by the data processing and analysis unit for cough assessment (5) process and analyse the data stored in the data storage unit (18).

11. The system for monitoring, characterisation and evaluation of a user's cough, according to any of the claims 7 and 8, **characterised by** comprising a central module (16) including the microcontroller (1) and
- at least one energy source (19); and
- at least one data transmission and reception unit (17), adapted to transmit the data resulting from the microcontroller's (1) operation, wherein the microcontroller (1) controls the data transmitted by the data transmission and reception unit (17), wherein the data is transmitted via a wired communication (21) or via a wireless communication protocol (22); and
at least one data storage unit (18), adapted to store the data resulting from the microcontroller's (1) operation, wherein the microcontroller (1) controls the data stored by the data storage unit (18);
and by the data processing and analysis unit for cough assessment (5) process and analyse the data transmitted by the data transmission and reception unit (17) and the data stored in the data storage unit (18).

12. The system for monitoring, characterisation and evaluation of a user's cough, according to any of the claims 1 to 6, **characterised by** comprising a central module (16) including the microcontroller (1) and
- at least one energy source (19); and
- at least one data transmission and reception unit (17), adapted to transmit the data obtained by the microcontroller (1), wherein the microcontroller (1) controls the data transmitted by the data transmission and reception unit (17), wherein the data is transmitted via a wired communication (21) or via a wireless communication protocol (22);
and by the microcontroller (1) comprises the data processing and analysis unit for cough assessment (5).

13. The system for monitoring, characterisation and evaluation of a user's cough, according to any of the claims 1 to 6, **characterised by** comprising a central module (16) including the microcontroller (1) and
- at least one energy source (19); and
- at least one data storage unit (18), adapted to store the data obtained by the microcontroller (1), wherein the microcontroller (1) controls the data stored by the data storage unit (18);
and by the microcontroller (1) comprises the data processing and analysis unit for cough assessment (5).

14. The system for monitoring, characterisation and evaluation of a user's cough, according to any of the claims 1 to 6, **characterised by** comprising a central module (16) including the microcontroller (1) and
- at least one energy source (19); and
- at least one data transmission and reception unit (17), adapted to transmit the data obtained by the microcontroller (1), wherein the microcontroller (1) controls the data transmitted by the data transmission and reception unit (17), wherein the data is transmitted via a wired communication (21) or via a wireless communication protocol (22); and
- at least one data storage unit (18), adapted to store the data obtained by the microcontroller (1), wherein the microcontroller (1) controls the data stored by the data storage unit (18);
and by the microcontroller (1) comprises the data processing and analysis unit for cough assessment (5).

15. The system for monitoring, characterisation and evaluation of a user's cough, according to any of the claims 9 to 14, **characterised by** the central module (16) comprises a screen (23), for example a touch screen, adapted to display information and/or to enter commands, such as monitoring pauses, and information, such as anthropometric information of its user, for the operation of the data processing and analysis unit for cough assessment (5).

16. The system for monitoring, characterisation and evaluation of a user's cough, according to any of the previous claims, **characterised by** the system is comprised in a patch (24).

17. The system for monitoring, characterisation and evaluation of a user's cough, according to any of the previous claims, **characterised by** the system is comprised in a water-resistant unit (26).

18. The system for monitoring, characterisation and evaluation of a user's cough, according to any of the previous claims, **characterised by** the microcontroller (1) comprises a sensory instrumentation unit malfunction detection unit (27).

19. A method for monitoring, characterisation and evaluation of a user's cough **characterised by** using the system as defined in any of the claims 1 to 18 and comprising the following steps:
i. Contact of at least one abdominal contraction sensor (3), comprised in the sensory instrumentation unit (2), with a user's body;
ii. Approach at least one acoustic sensor (4), comprised in the sensory instrumentation unit (2), to the user's body;
iii. Transmission of raw sensory signals from the active sensors to sensory instrumentation unit (2);
iv. Conditioning of said signals of said abdominal contraction sensor (3) and of said acoustic sensor (4), if active, by the sensory instrumentation unit (2);
v. Control of the acquisition of data obtained by the sensory instrumentation unit (2) by the microcontroller (1);
vi. Control of the activation of the acoustic sensor (4) by the microcontroller (1) only when an abdominal contraction is detected by said abdominal contraction sensor (3), wherein said acoustic sensor (4) only collects and transmits raw sensory signals to the sensory instrumentation unit (2) when it is activated;
vii. Reception of data from the data processing and analysis unit for cough assessment (5); and
viii. Monitoring, characterisation and evaluation of the user's cough by the data processing and analysis unit for cough assessment (5).

20. The method for monitoring, characterisation and evaluation of a user's cough, according to the previous claim, **characterised by** the sensory instrumentation unit (2) also receives raw sensory signals from a body positioning and motion characterisation sensor (9) and performing the conditioning of said sensory signals through the following additional steps:
i. Contact of the body positioning and motion characterisation sensor (9), comprised in a sensory instrumentation unit (2), with the user's body;
ii. Collection and transmission of raw sensory signals from the body positioning and motion characterisation sensor (9) to the sensory instrumentation unit (2);
and by the data processing and analysis unit for cough assessment (5) monitors, characterises, and evaluates additional cough patterns.

21. The method for monitoring, characterisation and evaluation of a user's cough, according to any of the claims 19 and 20, **characterised by** one or more external sensory instrumentation units (25) is connected to the system as defined in any of claims 1 to 17.

22. The method for monitoring, characterisation and evaluation of a user's cough, according to any of the claims 19 to 21, **characterised by** the system, as defined in any of claims 6 to 18, is synchronized with other devices.

23. The method for monitoring, characterisation and evaluation of a user's cough, according to any of the claims 19 to 22, **characterised by** the data processing and analysis unit for cough assessment (5) processes the data resulting from the microcontroller's (1) operation, using signal processing, machine learning and artificial intelligence algorithms.

24. The method for monitoring, characterisation and evaluation of a user's cough, according to any of the claims 19 to 22, **characterised by** the abdominal contraction sensor (3) is an EMG sensor, with a configuration of at least two electrodes, wherein the step of contacting the EMG sensor with the user's body comprises the following steps:
i. Contact an upper electrode of the surface electromyography sensor (7) with the user's body below the sternum xiphoid process, over the linea alba;
ii. Contact a lower electrode of the surface electromyography sensor (8) with the user's body on an imaginary line that includes the upper electrode of the surface electromyography sensor (7), parallel to the costal margin, in the epigastric region and over the rectus abdominis muscle;
iii. Optionally, contact an additional electrode in contact with the user's body on a bone dominant region, namely the iliac crest.

25. A portable device for monitoring, characterisation and evaluation of a user's cough **characterised by** comprising the system as defined in any of the claims 1 to 18, and a means for attachment to the user's body.

26. A computer apparatus **characterised by** comprising a processor configured to perform one or more steps of the method defined in any of the claims 19 to 24.

27. A computer program **characterised by** comprising instructions to enable the computer apparatus, as defined in the previous claim, to perform one or more steps of the method defined in any of the claims 19 to 24.

28. A means of reading through a computer apparatus **characterised by** comprising the installation of the computer program as defined in the previous claim.
